# EUROPEAN PATENT SPECIFICATION

(11) **EP 1 790 590 B1**
(45) Date of publication and mention of the grant of the patent: **20.05.2015**
(21) Application number: 06024524.8
(22) Date of filing: 27.11.2006
(51) Int. Cl.: B65F 1/16

(54) **Hospital waste containers and similar**
Behälter oder dergleichen für Krankenhausabfälle
Récipient ou similaires à déchets hospitaliers

(30) Priority: 25.11.2005 ES 200502925
(43) Date of publication of application: 30.05.2007
(73) Proprietor: EXPOPLASTI, S.L., 03440 IBI (ES)
(72) Inventor: Manzanares Perez, Manuel, 03008 Alicante (ES)
(74) Representative: Held, Stephan

(56) References cited:
- EP-A- 0 168 877
- EP-A- 0 808 782
- GB-A- 2 261 359

## Description

### Object of the Invention

The present invention relates to a hospital waste container comprising a body and a lid of the type used in hospital centers and the like, in which due to the nature of the content and once they have been used, it is necessary to arrange an inaccessible seal between the body of the container and the lid preventing handling said waste which can be hazardous for the handler.

The object of the invention is to obtain a hospital waste container facilitating the use thereof until it is filled, eliminating the risk of cuts and scratches for the staff handling it and considerably facilitating the closing operation.

### Background of the Invention

Waste involving a contamination risk for people is especially generated in certain hospital centers, therefore it requires a special handling until the time it is eliminated, generally by incineration.

To that end, containers are used which must allow their opening and closing as many times as it is necessary during the filling thereof, but once its elimination has been determined, they must allow an irremovable seal for its storage and transport to the incineration point, preventing the improper access to its interior to prevent the risks that may be derived from opening the container.

In this sense, ES 1 021 135 U must be emphasized, which utility model describes a container for hospital waste and the like, the body of which defines, at the level of its mouth, a perimetric edge framed by an also perimetric and outer groove whereas in the lid a perimetric groove is defined which can receive therein the perimetric edge of the body, with the placing of a sealing gasket, the outer wall of said groove having flanges penetrating in the slot or groove of the base body and traversing windows of the body thereof and which are locked when they go beyond said windows.

Although this solution is perfectly valid as regards the obtained results, i.e. as regards the sealing in the closure and the inviolability thereof, it has in contrast the fundamental drawback that the flanges of the lid constitute projecting elements involving a permanent risk of cuts and scratches for the staff handling the container, both for the hands and the latex gloves which are normally used as protective means, such that the accidental braking of one of said gloves can entail unpredictable consequences.

EP 0 808 782 A1 discloses a hospital waste container according to the preamble of claim 1 which is virtually an equivalent solution to that of the aforementioned utility model because it incorporates a virtually identical structure at the level of the lid, with the only exception that in this case the flanges are oriented outwards instead of inwards, with the peculiarity that at the level of the mouth, the body has a considerably narrower edge and outside it, it has a T-shaped partition also provided with slots or windows for the passage of the flanges. This solution keeps the flanges in the definitive closed situation for the container, but it still has the same aforementioned drawbacks during the operations for opening and closing the container before it is definitively closed as the mentioned utility model.

This patent also describes and claims a tool allowing opening said container after the definitive closing thereof, which is not very interesting as these containers have been provided with a single-use character, such that they are incinerated together with their content.

GB 2,261,359 A discloses another hospital waste container comprising a container base and a lid, the lid having several protrusions with a greater depth than the rest of the lid. The base is provided with corresponding locations in which the protrusions can be locked for closing the container base permanently. During closing the protrusions are pushed through plastic flap covering holes therefore causing a slight deformation of the flexible plastic flap which will then lock behind a base lid extending from a free end of the protrusion. The known container has the drawback that the flaps form sharp edges providing the risk of cuts and scratches by handling the container.

### Description of the Invention

The hospital waste container proposed by the invention in claim 1 solves in a fully satisfactory manner the drawbacks set forth above, such that there are no sharp edges either in the lid or on the body of the container which can constitute the slightest risk of cuts or scratches during, its handling.

To that end, each of the elastically deformable wings have on their outer face a recess intended to lock in their inside the corresponding anchoring clamp, such that said clamp is completely inaccessible for the user's hands, therefore it cannot constitute any damage risk for the latter either.

According to another embodiment of the invention, a silicone filling is applied to the lid which consequently remains associated to the latter.

### Description of the Drawings

To complement the description being made and for the purpose of better understanding the features of the invention according to a preferred practical embodiment thereof, a set of drawings is attached as an integral part of said description in which the following has been shown with an illustrative and nonlimiting character:
- Figure 1: shows a perspective exploded view of a hospital waste container and the like according to the object of the present invention.
- Figure 2: shows a sectional partial detail of the lid of the container.
- Figure 3: shows an outer perspective view of the lid shown in the previous figure.
- Figure 4: shows a detail similar to that of Figure 3, but corresponding to the mouth of the body of an embodiment different from the present invention.
- Figure 5: shows a sectional detail similar to that of Figure 2.

### Preferred Embodiment of the Invention

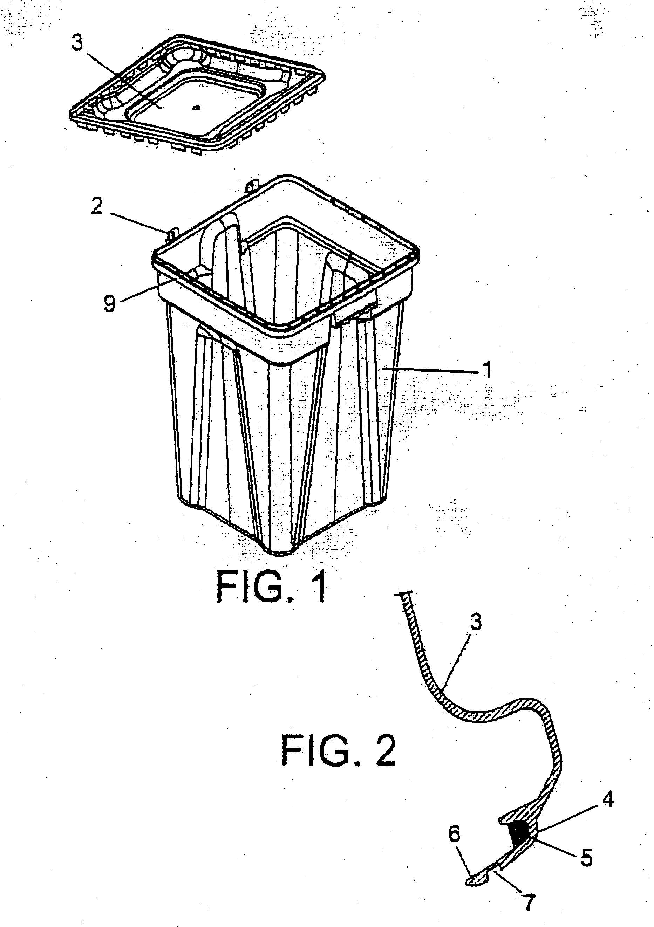

In view of the indicated figures and especially of Figure 1, it can be observed a hospital waste container and the like in which there is arranged a body (1), in an inverted truncated pyramid configuration for example, to which a lid (3) is joined in a hinged manner by means of hinges (2), such that said lid (3) can swing freely on the body (1) as many times as considered to be suitable, during the filling thereof, but when a downward pressure is applied on the entire perimeter thereof, a definitive and sealed blocking between the lid and the body occurs.

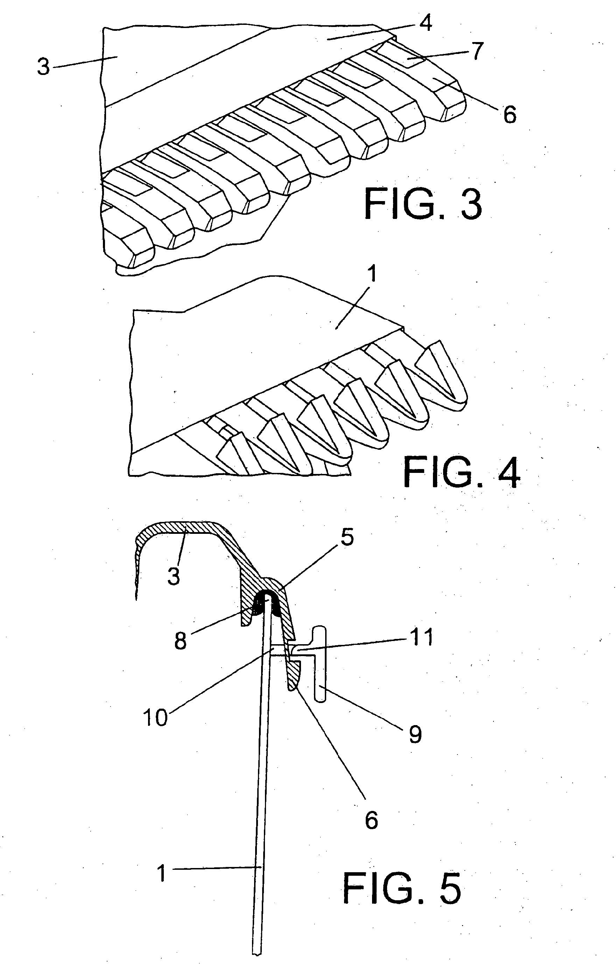

The lid (3) of the container incorporates a perimetric groove (4), with a downward-oriented mouth and an approximately V-shaped profile with a rounded vertex, which groove (4) is filled with a silicone cord or filling (5) so as to achieve the proper sealing in the closed situation shown in Figure 5.

A plurality of uniformly distributed wings (6) with rounded edges project from the outer partition of said groove (4), each of said wings (6) being provided in their outer face, as is especially observed in Figure 3, with a recess (7) having a rectangular prismatic configuration, intended for the definitive locking of the lid to the body.

In turn, the body (1), the mouth (8) of which is intended to deform the silicone filling (5), incorporates a ring (9) with a T-shaped profile relatively close to said mouth (8) and in its outer face, a plurality of uniformly distributed windows (10) being arranged in the horizontal branch of said ring, which windows coincide in number and position with the wings (6) of the lid and are intended to house the latter, carrying out the irremovable blocking thereof through its recesses (7), with the collaboration of clamps (11) defined in the outer edge of said windows (10) and with a suitable size to fit in the recesses (7) after the elastic deformation of the wings (4) and the subsequent recovery thereof.

In this manner and as observed in Figure 2, the means for locking the lid to the body are carried out in the mentioned recesses of said lid which are inaccessible for the user and cannot constitute the slightest damage risk therefore, whereas the complementary means arranged in the body, i.e. the clamps (11) are also located inside the ring (9), in a completely inaccessible situation and consequently in a situation of total absence of risk for the person handling the container.

## Claims

1. A hospital waste container comprising a body (1) and a lid (3), wherein the lid (3) incorporates a perimetric groove (4) with a downward-oriented mouth inside of which the mouth (8) of the body (1) of the container fits, wherein an outer wall of the groove (4) defined in the free edge of the lid (3) incorporates a plurality of uniformly distributed elastically deformable wings (6) extending substantially downwards and having rounded edges, whereas the body (1) incorporates at the level of its mouth (8) and also externally a ring (9) with a T-shaped cross-section, in the horizontal branch of which there are defined windows (10) which coincide in number and position with fhe wings (6) of the lid (3) so as to allow the passage of the latter, wherein anchoring clamps (11) are arranged in each of said windows (10) in the outer edge thereof, **characterized in that** each of the wings (6) is provided in its outer face with a recess (7) and that each anchoring clamp (11) can be locked in the recess (7) of the corresponding wing (6) after the deformation and subsequent elastic recovery thereof.

2. A hospital waste container according to claim 1, **characterized in that** the groove (4) defined in the free edge of the lid (3) incorporates a silicon filling (5) adapted to be seated on the mouth (8) of the body (1) of the container.

## Patentansprüche

1. Krankenhausabfallbehälter, der einen Körper (1) und einen Deckel (3) umfasst, wobei der Deckel (3) eine Umfangsnut (4) mit einer nach unten orientierten Öffnung, in die die Öffnung (8) des Körpers (1) des Behälters passt, aufweist, wobei eine Außenwand der Nut (4), die in der freien Kante des Deckels (3) definiert ist, mehrere gleichmäßig verteilte, elastisch verformbare Flügel (6) aufweist, die sich im Wesentlichen nach unten erstrecken und abgerundete Kanten besitzen, während der Körper (1) auf der Höhe seiner Öffnung (8) und ebenfalls außen einen Ring (9) mit einem T-förmigen Querschnitt aufweist, in dessen horizontalem Schenkel Fenster (14) definiert sind, deren Anzahl und deren Position mit den Flügeln (6) des Deckels (3) übereinstimmen, um den Durchgang der Letzteren zuzulassen, wobei in jedem der Fenster (10) in deren Außenkante Verankerungsklemmen (11) angeordnet sind,
**dadurch gekennzeichnet, dass** jeder der Flügel (6) in seiner äußeren Fläche mit einer Aussparung (7) versehen ist und das jede Verankerungsklemme (11) in der Aussparung (7) des entsprechenden Flügels (6) verriegelt werden kann, nachdem er verformt worden ist und anschließend seine ursprüngliche Form wieder erlangt hat.

2. Krankenhausabfallbehälter nach Anspruch 1, **dadurch gekennzeichnet, dass** die in der freien Kante des Deckels (3) definierte Nut (4) eine Silikonfüllung (5) aufweist, die dafür ausgelegt ist, an der Öffnung (8) des Körpers (1) des Behälters zu sitzen.

## Revendications

1. Contenant de déchets hospitaliers comprenant un corps (1) et un couvercle (3), dans lequel le couvercle (3) incorpore une rainure périmétrique (4) avec une embouchure orientée vers le bas à l'intérieur de laquelle s'ajuste l'embouchure (8) du corps (1) du contenant, dans lequel une paroi extérieure de la rainure (4) définie dans le bord libre du couvercle (3) incorpore une pluralité d'ailettes pouvant être déformées de manière élastique et réparties de manière uniforme (6), qui s'étendent sensiblement vers le bas et qui présentent des bords arrondis, tandis que le corps (1) incorpore, au niveau de son embouchure (8) et également de manière extérieure un anneau (9) qui présente une section transversale en forme de T, dans la branche duquel sont définies des fenêtres (10) dont le nombre et la position coïncident avec ceux des ailettes (6) du couvercle (3), de façon à permettre le passage de ce dernier, dans lequel sont agencées des pinces d'ancrage (11) dans chacune desdites fenêtres (10) dans le bord extérieur de celles-ci, **caractérisé en ce que** chacune des ailettes (6) est dotée d'un renfoncement (7) dans sa face extérieure, et **en ce que** chaque pince d'ancrage (11) peut être verrouillée dans le renfoncement (7) de l'ailette correspondante (6) après la déformation et la récupération élastique ultérieure de celle-ci.

2. Contenant de déchets hospitaliers selon la revendication 1, **caractérisé en ce que** la rainure (4) définie dans le bord libre du couvercle (3) incorpore un matériau de remplissage de silicone (5) adapté de façon à être placé sur l'embouchure (8) du corps (1) du contenant.
